# NOUVEAU FASCICULE DE BREVET EUROPEEN

(11) **EP 2 793 831 B2**
(45) Date de publication et mention de la décision concernant l'opposition: **10.11.2021**
(45) Mention de la délivrance du brevet: 03.10.2018
(21) Numéro de dépôt: 12819093.1
(22) Date de dépôt: 19.12.2012
(51) Int. Cl.: A61K 8/46, A61Q 5/12, A61K 8/34, A61K 8/37, A61K 8/92, A61K 8/85, A61K 8/44

(54) **PROCÉDÉ DE TRAITEMENT COSMÉTIQUE AVEC UNE COMPOSITION COMPRENANT UN TENSIOACTIF PROCÉDÉ ANIONIQUE, UN ALCOOL GRAS SOLIDE ET UN ESTER GRAS SOLIDE**
KOSMETISCHE BEHANDLUNG MIT EINER ZUSAMMENSETZUNG ENTHALTEND EIN ANIONISCHES TENSID, EINEN FESTEN FETTALKOHOL UND EINEN FESTEN FETTESTER
COSMETIC TREATMENT PROCESS WITH A COMPOSITION COMPRISING AN ANIONIC SURFACTANT, A SOLID FATTY ALCOHOL AND A SOLID FATTY ESTER

(30) Priorité: 20.12.2011 FR 1162038; 20.12.2011 FR 1162037; 03.01.2012 US 201261582741 P; 03.01.2012 US 201261582733 P
(43) Date de publication de la demande: 29.10.2014
(73) Titulaire: L'Oréal, 75008 Paris (FR)
(72) Inventeur: MATHONNEAU, Estelle, 93400 Saint-Ouen (FR); FACK, Géraldine, F-92300 Levallois (FR); CHESNEAU, Laurent, F-92300 Levallois Perret (FR); LE CHAUX, Virginie, F-95130 Franconville (FR)
(74) Mandataire: L'Oreal
(86) Numéro de dépôt international: PCT/FR2012/052981
(87) Numéro de publication internationale: WO 2013/093332

(56) Documents cités:
- EP-A1- 0 555 690
- EP-A1- 1 637 121
- WO-A1-2008/055816
- WO-A1-2010/046048
- WO-A1-2010/052092
- WO-A1-2010/052093
- WO-A1-2010/106044
- WO-A1-2010/149604
- WO-A1-2011/000488
- WO-A1-2011/004847
- WO-A1-2011/047421
- WO-A2-2009/015913
- WO-A2-2010/149423
- WO-A2-2011/000489
- WO-A2-2011/092083
- DE-A1- 2 707 814
- DE-A1-102007 017 434
- DE-A1-102007 036 499
- DE-A1-102008 052 521
- DE-A1-102009 031 280
- DE-A1-102011 013 341
- US-A- 5 656 262
- US-A1- 2003 086 897
- US-A1- 2003 165 551
- US-A1- 2003 206 934
- US-A1- 2004 136 941
- US-A1- 2004 141 936
- US-A1- 2004 228 824
- US-A1- 2005 002 879
- US-A1- 2005 037 036
- US-A1- 2006 008 426
- US-A1- 2007 297 992
- US-A1- 2009 111 750
- US-A1- 2010 028 275
- US-A1- 2011 165 108
- US-B1- 6 419 938
- US-B1- 6 551 601
- US-B2- 7 993 631
- Rahn Cosmetics: "Haut- und Haarpflege im Zeichen der Naturkosmetik", , 1 janvier 2010 (2010-01-01), pages 1-4, XP055045113, Extrait de l'Internet: URL:http://www.rahn-group.com/file_uploads /bibliothek/k_228_Cosmetics/k_467_Spotligh t/2898_0_aquarich_eco_spotlight_de.pdf [extrait le 2012-11-21]
- Anonymous: "Hand Cream ( Mades Signature)", Mintel, 1667957, November 2011 (2011-11), pages 1-3,
- Anonymous: "Face Cream for Children' (DM Alverde Naturkosmetik)", MINTEL Database, 1664755, November 2011 (2011-11), pages 1-3,
- Anonymous: "Aminoeffectivesleep Cream (VECUA Skinperfectsphere)", MINTEL, 1172804, September 2009 (2009-09), pages 1-3,
- Anonymous: "Radiance-Boosting Fluid Day Cream (Daniel Jouvance Physiomarine Beaute)", MINTEL, 1033094, January 2009 (2009-01), pages 1-4,
- Anonymous: "Soin de Nuit Recuperateur Jeunesse Night Cream (Supraceane)", MINTEL, 297381, August 2004 (2004-08), pages 1-2,
- Anonymous: "Desert Bloom Intense Hand Rescue (Molton Brown Body Remedies Hydrate)", MINTEL, 1620270, August 2011 (2011-08), pages 1-4,
- Anonymous: "Repair Conditioner (DM Alverde Naturkosmetik)", MINTEL, 125534, January 2010 (2010-01), pages 1-3,
- ANONYMOUS: "High-Spreading Ester Emollient for AP /Deo Concepts", RESEARCH DISCLOSURE, vol. 510, no. 13, 510013, October 2006 (2006-10-01)
- "Neue Emulgatoren für sprühbare kosmetische Zubereitungen", Research Disclosure, Kenneth Mason Publications, Hampshire, UK, GB, vol. 508, no. 54, 1 August 2006 (2006-08-01), page 1043, GB ISSN: 0374-4353
- Disclosed anonymously: "Neue emulgatoren für kosmetische AP/Deo zubereitungen", Research Disclosure, Kenneth Mason Publications, Hampshire, UK, GB, vol. 510, no. 60, 1 October 2006 (2006-10-01), page 1347, GB ISSN: 0374-4353

## Description

La présente invention concerne un procédé de traitement cosmétique, notamment un procédé de conditionnement, des fibres kératiniques humaines, comprenant au moins un tensioactif anionique particulier, au moins un alcool gras solide et au moins un ester gras solide de monoalcool.

Dans le domaine des conditionneurs, on emploie généralement des agents de conditionnement qui peuvent être un tensioactif cationique, un polymère cationique, une silicone, une huile, un corps gras ou un de leurs mélanges. Ces agents de conditionnement sont utilisés pour améliorer le démêlage et la douceur des cheveux, qu'ils soient mouillés ou séchés, mais peuvent avoir tendance à alourdir la chevelure et à la ternir.
Il en résulte que l'utilisation d'agents conditionneurs insolubles est fortement limitée d'une part du fait des difficultés de stabilisation des compositions les comprenant, d'autre part, du fait des inconvénients cosmétiques en terme d'alourdissement, de charge et de regraissage des matières kératiniques liés à des dispersions grossières ou hétérogènes. Les agents de conditionnement insolubles, en particulier les alcools gras, sont connus et utilisés dans les compositions capillaires comme notamment dans les documents JP2002-20791, JP9-30938, US2009/005449 et US2009/005460. On connait encore WO2010/149604 qui concerne une composition capillaire comprenant une fraction protéique anio-nique et un tensioactif comprenant des groupes alkyle en C16-C22, un composé gras en C16-C22 et de l'eau. Cependant, ces compositions ne présentent pas des performances cosmétiques de haut niveau en terme de démêlage et de lissage, sans charge ni alourdissement de la chevelure et en maintenant le niveau adéquat des qualités d'usage.

La demanderesse a maintenant découvert que l'utilisation decompositions comprenant au moins un tensioactif anionique particulier, au moins un alcool gras solide, et au moins un ester gras solide permettait d'obtenir une composition ayant de très bonnes propriétés cosmétiques ; en particulier le tissage, la douceur, la souplesse, la brillance sont améliorés, notamment sur les cheveux sensibilisés. Dans le cas des cheveux frisés et/ou crépus, on observe également une diminution du volume permettant un meilleur contrôle de la chevelure.
On a également constaté que les compositions selon l'invention pouvaient également apporter une protection de la couleur aux lavages des cheveux artificiellement colorés.

L'invention a donc pour objet un procédé de traitement cosmétique, notamment de conditionnement, des fibres kératiniques humaines, et tout spécialement des cheveux, voire des cheveux sensibilisés, dans lequel on applique sur lesdites fibres kératiniques, une composition cosmétique non colorante et non oxydante comprenant :
- un ou plusieurs tensioactifs anioniques sulfates, sulfonates ou carboxyliques, tels qu'au moins 50% en poids d'entre eux comportent des chaînes grasses ayant un nombre d'atomes de carbone supérieur ou égal à 14 ; présents dans la composition en une quantité totale allant de 0,5 à 10% en poids, par rapport au poids total de la composition ;
   les tensioactifs anioniques sulfates, sulfonates ou carboxyliques étant choisis parmi les sels des types suivants : les alkylsulfates, les alkyléthersulfates, les alkylamidoéthersulfates, les alkylarylpolyéthersulfates, les monoglycéride-sulfates; les alkylsulfonates, les alkylamidesulfonates, les alkylarylsulfonates, les alpha-oléfine-sulfonates, les paraffine-sulfonates, les alkylsulfoacétates, les groupes alkyle et acyle de tous ces composés comportant de préférence de 14 à 30 atomes de carbone, mieux de 16 à 22 atomes de carbone, et le groupe aryle désignant de préférence un groupe phényle ou benzyle;
- un ou plusieurs alcools gras solides, présents en une quantité allant de 4 à 10% en poids, par rapport au poids total de la composition, et
- un ou plusieurs esters gras solides de monoalcools,
et on effectue optionnellement un rinçage après un éventuel temps de pose.

Dans la présente description, l'expression "au moins un" est équivalente à l'expression "un ou plusieurs".

Par composition non colorante, on entend selon la présente invention, une composition ne contenant pas de colorant des fibres kératiniques tels que les colorants directs ou les précurseurs de colorant d'oxydation (bases et coupleurs). S'ils sont présents, leur teneur ne dépasse pas 0,005% en poids par rapport au poids total de la composition. En effet, à une telle teneur, seule la composition serait teintée, c'est-à-dire qu'on n'observerait pas d'effet de coloration des fibres kératiniques.

Par composition non oxydante, on entend selon la présente invention, une composition ne contenant pas d'agent oxydant habituellement utilisé dans les traitements des fibres kératiniques tels que le peroxyde d'hydrogène, le peroxyde d'urée, les bromates ou ferricyanures de métaux alcalins, les sels peroxygénés comme par exemple les persulfates, les perborates, les peracides et leurs précurseurs, les percarbonates de métaux alcalins ou alcalino-terreux et les peracides et leurs précurseurs. S'ils sont présents, leur teneur ne dépasse pas 0,005% en poids par rapport au poids total de la composition.

Dans la présente description, on qualifie une entité comme étant "anionique" lorsqu'elle possède au moins une charge négative permanente ou lorsqu'elle peut être ionisée en une entité chargée négativement, dans les conditions d'utilisation des compositions de l'invention (milieu, pH par exemple) et ne comprenant pas de charge cationique.

### Tensioactifs anioniques

Les tensioactifs anioniques sulfate(s) ou sulfonate(s) selon l'invention sont des tensioactifs anioniques comportant au moins une fonction sulfate (-OSO₃H ou-OSO₃⁻) et/ou une fonction sulfonate (-SO₃H ou -SO₃⁻).
Les tensioactifs anioniques carboxyliques selon l'invention sont des tensioactifs anioniques comportant au moins une fonction carboxylique -COOH ou -COO⁻,

Il est rappelé que lesdits tensioactifs anioniques sulfates, sulfonates ou carboxyliques sont tels qu'au moins 50% en poids d'entre eux comportent des chaînes grasses ayant un nombre d'atomes de carbone supérieur ou égal à 14.

Les tensioactifs anioniques sulfates ou sulfonates pouvant être utilisés dans la composition selon l'invention sont choisis parmi les sels, en particulier les sels de métaux alcalins tels que les sels de sodium, les sels d'ammonium, les sels d'amines, les sels d'aminoalcools ou les sels de métaux alcalino-terreux, par exemple, de magnésium, des types suivants : les alkylsulfates, les alkyléthersulfates, les alkylamidoéthersulfates, les alkylarylpolyéthersulfates, les monoglycéride-sulfates; les alkylsulfonates, les alkylamidesulfonates, les alkylarylsulfonates, les alpha-oléfine-sulfonates, les paraffine-sulfonates, les alkylsulfo-acétates, les groupes alkyle et acyle de tous ces composés comportant de préférence de 14 à 30 atomes de carbone, mieux de 16 à 22 atomes de carbone, et le groupe aryle désignant de préférence un groupe phényle ou benzyle.
De préférence, les tensioactifs anioniques sulfates ou sulfonates sont choisis parmi les alkylsulfates en C16-C18 et les alkyléthersulfates en C16-18 et leurs mélanges, en particulier sous forme de sels de métaux alcalins ou alcalino-terreux, d'ammonium, d'amine ou d'aminoalcool.

Le ou les tensioactifs anioniques selon l'invention sont présents dans la composition en une quantité totale allant de 0,5 à 10% en poids, mieux encore de 1 à 5% en poids, par rapport au poids total de la composition.

Les "alcools gras solides" sont solides à température ambiante (25°C) et à pression atmosphérique (780 mmHg ou 1 atm); ils sont insolubles dans l'eau, c'est-à-dire qu'ils présentent une solubilité dans l'eau inférieure à 1 % en poids et de préférence inférieure à 0,5% en poids.

On entend par alcool gras, un alcool aliphatique à longue chaine comprenant de 8 à 40 atomes de carbone, de préférence de 12 à 34, voire de 12 à 30, atomes de carbone, et comprenant au moins un groupe hydroxyle OH. Ces alcools gras ne sont ni oxyalkylénés, ni glycérolés.

De préférence les alcools gras solides sont de structure R-OH avec R désignant un groupe alkyle linéaire, éventuellement substitué par un ou plusieurs groupes hydroxy, comprenant de 12 à 40, mieux de 12 à 34, voire de 12 à 30, et tout préférentiellement de 12 à 24, atomes de carbone.

Les alcools gras solides susceptibles d'être employés dans le cadre de l'invention sont plus particulièrement choisis parmi :
- l'alcool laurique ou laurylique, (1 dodécanol) ;
- l'alcool myristique ou myristylique, (1-tétradécanol) ;
- l'alcool cétylique, (1-hexadécanol) ;
- l'alcool stéarylique, (1-octadécanol) ;
- l'alcool arachidylique, (1-eicosanol) ;
- l'alcool behenylique, (1-docosanol) ;
- l'alcool lignocérylique, (1-tetracosanol) ;
- l'alcool cerylique, (1-hexacosanol) ;
- l'alcool montanylique, (1-octacosanol) ;
- l'alcool myricylique, (1-triacontanol) ;
   et leurs mélanges.

Plus particulièrement l'alcool gras solide est choisi parmi l'alcool cétylique, l'alcool stéarylique, l'alcool béhénylique et leurs mélanges tels que l'alcool cétylstéarylique ou cétéarylique.
Les alcools gras peuvent être des mélanges, ce qui signifie que, dans un produit commercial par exemple, peuvent coexister plusieurs espèces notamment de longueur de chaînes différente, sous forme d'un mélange.

Le ou les alcools gras solides selon l'invention sont présents dans la composition en une quantité allant de 4 à 10% en poids, par rapport au poids total de la composition.

De préférence, le rapport pondéral alcool(s) gras solide(s) / tensioactif(s) anionique(s) selon l'invention va de 1 à 15, mieux de 2 à 10.

### Esters gras solides

La composition utilisée dans le procédé selon l'invention comprend en outre un ou plusieurs esters gras solides à température ambiante (25°C) et à pression atmosphérique (1 atm).
Les esters gras solides sont des esters de monoalcools, notamment de monoalcools comportant au moins 10 atomes de carbone, encore mieux de monoalcools saturés comportant au moins 10 atomes de carbone.
Préférentiellement, les esters gras solides sont des esters d'acides carboxyliques saturés comportant au moins 10 atomes de carbone, et de monoalcools saturés comportant au moins 10 atomes de carbone.
Les acides carboxyliques et/ou les monoalcools saturés peuvent être linéaires ou ramifiés.
Les acides carboxyliques saturés comportent de préférence de 10 à 30 atomes de carbone et plus particulièrement de 12 à 24 atomes de carbone. Ils peuvent être éventuellement hydroxylés.
Les monoalcools saturés comportent de préférence de 10 à 30 atomes de carbone et plus particulièrement de 12 à 24 atomes de carbone.
De préférence, les esters gras solides sont choisis parmi les myristates de myristyle, de cétyle ou de stéaryle, les palmitates de myristyle, de cétyle ou de stéaryle, les stéarates de myristyle, de cétyle ou de stéaryle, ainsi que leurs mélanges.

Le ou lesdits esters gras solides sont de préférence présents dans la composition en une quantité comprise entre 0,1 et 10% en poids, de préférence entre 0,5 et 5% en poids, par rapport au poids total de la composition.

### Corps gras liquides

La composition utilisée dans le procédé selon l'invention peut comprendre en outre un ou plusieurs corps gras liquides.

Par corps gras, on entend un composé organique insoluble dans l'eau à température ambiante (25° C) et pression atmosphérique (760 mm de Hg), c'est-à-dire dont la solubilité est inférieure à 5% et de préférence à 1 % encore plus préférentiellement à 0,1%.
Ils présentent généralement dans leur structure au moins une chaîne hydrocarbonée comportant au moins 6 atomes de carbone et/ou un enchainement d'au moins deux groupements siloxane. En outre, les corps gras sont généralement solubles dans les solvants organiques dans les mêmes conditions de température et de pression, comme par exemple le chloroforme, l'éthanol, le benzène, l'huile vaseline ou le décaméthyl cyclopentasiloxane. Ces corps gras ne sont ni polyoxyéthylénés ni polyglycérolés.
Les corps gras selon l'invention sont liquides à température ambiante (25°C) et pression atmosphérique (780 mmHg).

Ils sont de préférence choisis parmi les alcools gras liquides, les esters gras liquides, les huiles de silicone, les hydrocarbures en C6-C16, les hydrocarbures à plus de 16 atomes de carbone, les huiles non siliconées d'origine animale, les huiles végétales de type triglycérides, les triglycérides synthétiques, les huiles fluorées, et leurs mélanges.

Les alcools gras liquides, en particulier ceux en C₁₀-C₃₄ présentent des chaînes carbonées ramifiées ou possèdent une ou plusieurs (de préférence 1 à 3) insaturations.
Ils sont de préférence ramifiés et/ou insaturés, et comportent de 12 à 40 atomes de carbone. Ils sont non oxyalkylénés et non glycérolés.
Les alcools gras liquides présentent de préférence la structure R-OH, dans laquelle R désigne un groupement alkyle ramifié en C₁₂-C₂₄ ou alkényle en C₁₂-C₂₄, R pouvant être substitué par un ou plusieurs groupements hydroxy. De préférence, R ne contient pas de groupement hydroxy.
De préférence, l'alcool gras liquide est un alcool saturé ramifié.
A titre d'exemple, on peut citer l'alcool oléique, l'alcool linoléique, l'alcool linolénique, l'alcool isocétylique, l'alcool isostéarylique, le 2-octyl-1-dodécanol, le 2-butyl octanol, le 2-hexyl-1-décanol, le 2-decyl-1-tétradécanol, le 2-tétradécyl-1-cétanol et leurs mélanges. Préférentiellement, l'alcool gras liquide est le 2-octyl 1-dodécanol.
Les alcools gras liquides peuvent être des mélanges, ce qui signifie que dans un produit commercial peuvent coexister plusieurs espèces notamment de longueur de chaînes différente, sous forme d'un mélange.

Les esters gras liquides susceptibles d'être utilisés peuvent être des esters de monoalcools ou de polyols avec des mono- ou des polyacides, au moins un des alcools et/ou des acides comportant au moins une chaîne de plus de 7 atomes de carbones. De préférence, l'ester gras liquide est choisi parmi les esters d'acide gras et de monoalcool. De préférence, l'un au moins des alcools et/ou acides est ramifié.
A titre d'exemple, on peut citer le myristate d'isopropyle, le palmitate d'isopropyle, l'isononanoate d'isononyle, le palmitate de 2-éthylhexyle, le laurate de 2-hexyldécyle, le palmitate de 2-octyldécyle, le myristate de 2-octyldodécyle; et leurs mélanges.

Les silicones utilisables conformément à l'invention se présentent sous forme d'huiles.
De préférence, la silicone est choisie parmi les polydialkylsiloxanes, notamment les polydiméthylsiloxanes (PDMS), et les polysiloxanes organomodifiés comportant au moins un groupement fonctionnel choisi parmi les groupements aminés et les groupements alcoxy. Les organopolysiloxanes sont définis plus en détail dans l'ouvrage de Walter NOLL "Chemistry and Technology of Silicones" (1968), Academie Press. Elles peuvent être volatiles ou non volatiles.

Lorsqu'elles sont volatiles, les silicones sont plus particulièrement choisies parmi celles possédant un point d'ébullition compris entre 60°C et 260°C, et plus particulièrement encore parmi:
- (i) les polydialkylsiloxanes cycliques comportant de 3 à 7, de préférence de 4 à 5 atomes de silicium. Il s'agit, par exemple, de l'octaméthylcyclotétrasiloxane commercialisé notamment sous le nom de VOLATILE SILICONE® 7207 par UNION CARBIDE ou SILBIONE® 70045 V2 par RHODIA, le décaméthylcyclopentasiloxane commercialisé sous le nom de VOLATILE SILICONE® 7158 par UNION CARBIDE, et SILBIONE® 70045 V5 par RHODIA, ainsi que leurs mélanges.
   On peut également citer les cyclocopolymères du type diméthylsiloxanes/ méthylalkylsiloxane, tel que la SILICONE VOLATILE® FZ 3109 commercialisée par la société UNION CARBIDE, de formule : 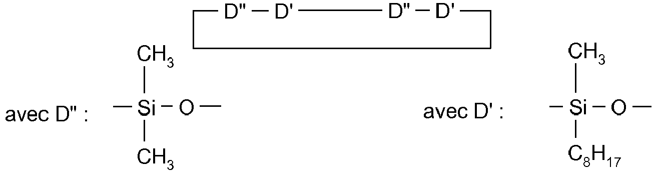 On peut également citer les mélanges de polydialkylsiloxanes cycliques avec des composés organiques dérivés du silicium, tels que le mélange d'octaméthylcyclotétrasiloxane et de tétratriméthylsilylpentaérythritol (50/50) et le mélange d'octaméthylcyclotétrasiloxane et d'oxy-1,1'-(hexa-2,2,2',2',3,3'-triméthylsilyloxy) bis-néopentane ;
- (ii) les polydialkylsiloxanes volatiles linéaires ayant 2 à 9 atomes de silicium et présentant une viscosité inférieure ou égale à 5.10-6m²/s à 25° C. Il s'agit, par exemple, du décaméthyltétrasiloxane commercialisé notamment sous la dénomination "SH 200" par la société TORAY SILICONE. Des silicones entrant dans cette classe sont également décrites dans l'article publié dans Cosmetics and Toiletries, Vol. 91, Jan. 76, P. 27-32 - TODD & BYERS "Volatile Silicone fluids for cosmetics".

On utilise de préférence des polydialkylsiloxanes non volatiles, des polyorganosiloxanes modifiés par les groupements organofonctionnels ci-dessus ainsi que leurs mélanges.
Ces silicones sont plus particulièrement choisies parmi les polydialkylsiloxanes parmi lesquels on peut citer principalement les polydiméthylsiloxanes à groupements terminaux triméthylsilyl. La viscosité des silicones est mesurée à 25°C selon la norme ASTM 445 Appendice C.
Parmi ces polydialkylsiloxanes, on peut citer à titre non limitatif les produits commerciaux suivants : les huiles SILBIONE® des séries 47 et 70 047 ou les huiles MIRASIL® commercialisées par RHODIA telles que, par exemple l'huile 70 047 V 500 000; les huiles de la série MIRASIL® commercialisées par la société RHODIA; les huiles de la série 200 de la société DOW CORNING telles que la DC200 ayant viscosité 60 000 mm²/s; les huiles VISCASIL® de GENERAL ELECTRIC et certaines huiles des séries SF (SF 96, SF 18) de GENERAL ELECTRIC.
On peut également citer les polydiméthylsiloxanes à groupements terminaux diméthylsilanol connus sous le nom de dimethiconol (CTFA), tels que les huiles de la série 48 de la société RHODIA.
Dans cette classe de polydialkylsiloxanes, on peut également citer les produits commercialisés sous les dénominations "ABIL WAX® 9800 et 9801" par la société GOLDSCHMIDT qui sont des polydialkyl (C1-C20) siloxanes.
Des produits plus particulièrement utilisables conformément à l'invention sont des mélanges tels que les mélanges formés à partir d'un polydiméthylsiloxane hydroxylé en bout de chaîne, ou diméthiconol (CTFA) et d'un polydiméthylsiloxane cyclique également appelé cyclométhicone (CTFA) tel que le produit Q2-1401 commercialisé par la société DOW CORNING.

Les silicones organomodifiées utilisables conformément à l'invention sont des silicones telles que définies précédemment et comportant dans leur structure un ou plusieurs groupements organofonctionnels fixés par l'intermédiaire d'un groupe hydrocarboné.
Outre les silicones décrites ci-dessus, les silicones organomodifiées peuvent être des polydiarylsiloxanes, notamment des polydiphénylsiloxanes, et des polyalkylaryl siloxanes fonctionnalisés parles groupes organo fonctionnels mentionnés précédemment.
Les polyalkylarylsiloxanes sont particulièrement choisis parmi les polydiméthyl/méthylphénylsiloxanes, les polydiméthyl /diphénylsiloxanes linéaires et/ou ramifiés de viscosité allant de 1.10⁻⁵ à 5.10⁻²m²/s à 25°C.
Parmi ces polyalkylarylsiloxanes, on peut citer à titre d'exemple les produits commercialisés sous les dénominations suivantes : les huiles SILBIONE® de la série 70 641 de RHODIA; les huiles des séries RHODORSlL® 70 633 et 763 de RHODIA; l'huile DOW CORNING 556 COSMETIC GRAD FLUID de DOW CORNING; les silicones de la série PK de BAYER comme le produit PK20; les silicones des séries PN, PH de BAYER comme les produits PN1000 et PH1000 ; certaines huiles des séries SF de GENERAL ELECTRIC telles que SF 1023, SF 1154, SF 1250, SF 1265.

Parmi les silicones organomodifiées, on peut citer les polyorganosiloxanes comportant : (i) des groupements aminés substitués ou non comme les produits commercialisés sous la dénomination GP 4 Silicone Fluid et GP 7100 par la société GENESEE; les groupements aminés substitués étant en particulier des groupements aminoalkyle en C1-C4 ; (ii) des groupements alcoxylés, comme le produit commercialisé sous la dénomination "SILICONE COPOLYMER F-755" par SWS SILICONES.
Dans une variante de l'invention, les silicones ne sont pas organomodifiées.

Les hydrocarbures en C6-C16 sont de préférence linéaires ou ramifiés, éventuellement cycliques, et sont de préférence des alcanes. On peut citer l'hexane, le dodécane, les isoparaffines comme l'isohexadécane, l'isodécane.

A titre d'huiles hydrocarbonées d'origine animale, on peut citer le perhydrosqualène.
Les huiles triglycérides d'origine végétale ou synthétique sont choisies de préférence parmi les triglycérides liquides d'acides gras comportant de 6 à 30 atomes de carbone, comme les triglycérides des acides heptanoïque ou octanoïque ou encore, parexemple les huiles de tournesol, de maïs, de soja, de courge, de pépins de raisin, de sésame, de noisette, d'abricot, de macadamia, d'arara, de tournesol, de pracaxi, d'argan, de ricin, d'avocat, les triglycérides des acides caprylique/caprique comme ceux vendus par la société Stearineries Dubois ou ceux vendus sous les dénominations Miglyol® 810, 812 et 818 par la société Dynamit Nobel, l'huile de jojoba, l'huile de beurre de karité.

Les hydrocarbures linéaires ou ramifiés, d'origine minérale ou synthétique, de plus de 16 atomes de carbone, sont choisis de préférence parmi les huiles de paraffine, la vaseline, l'huilede vaseline, les polydécènes, le polyisobutène hydrogéné tel que Parléam®.

Les huiles fluorées peuvent être choisies parmi le perfluorométhylcyclopentane et le perfluoro-1,3 diméthylcyclohexane, vendus sous les dénominations de "FLUTEC® PC1" et "FLUTEC® PC3" par la Société BNFL Fluorochemicals ; le perfluoro-1,2-diméthylcyclobutane; les perfluoroalcanes tels que le dodécafluoropentane et le tétradécafluorohexane, vendus sous les dénominations de "PF 5050®" et "PF 5060®" par la Société 3M, ou encore le bromoperfluorooctyle vendu sous la dénomination "FORALKYL®" parla Société Atochem ; le nonafluorométhoxybutane et le nonafluoroéthoxyisobutane ; les dérivés de perfluoromorpholine, tels que la 4-trifluorométhyl perfluoromorpholine vendue sous la dénomination "PF 5052®" par la Société 3M.

De préférence, les corps gras liquides sont choisis parmi les huiles de silicone et les alcools gras liquides, et leurs mélanges. Préférentiellement, les corps gras liquides sont choisis parmi les huiles de silicone.

Lorsqu'ils sont présents, les corps gras liquides sont de préférence présents en une quantité allant de 0,01 à 20% en poids, préférentiellement de 0,1 à 10% en poids, et mieux encore de 0,5 à 5% en poids, par rapport au poids total de la composition.

### Autres ingrédients

La composition cosmétique utilisée dans le procédé selon l'invention peut également comprendre en outre au moins un tensioactif non ionique et/ou au moins un tensioactif amphotère.

Les tensioactifs non ioniques susceptibles d'être utilisés sont des composés bien connus; on peut voir notamment à cet égard "Handbook of Surfactants" par M.R. PORTER, éditions Blackie & Son (Glasgow and London), 1991, pp 116-178. Ils sont choisis notamment parmi les alcools, les alpha-diols, les alkyl(C₁₋₂₀)phénols, ces composés étant polyéthoxylés, polypropoxylésou polyglycérolés, et ayant une chaîne grasse comportant, par exemple, de 8 à 30 atomes de carbone, de préférence de 8 à 22, le nombre de groupements oxyde d'éthylène ou oxyde de propylène pouvant aller notamment de 2 à 50 et le nombre de groupements glycérol pouvant aller notamment de 2 à 30.
On peut également citer les condensats d'oxyde d'éthylène et d'oxyde de propylène sur des alcools gras; les amides gras polyéthoxylés ayant de préférence de 2 à 30 motifs d'oxyde d'éthylène, les amides gras polyglycérolés comportant en moyenne de 1 à 5 groupements glycérol et en particulier de 1,5 à 4, les esters d'acides gras du sorbitane polyéthoxylés ayant de préférence de 2 à 40 motifs d'oxyde d'éthylène, les esters d'acides gras du saccharose, les esters d'acides gras du polyéthylèneglycol, les (alkyl en C₆₋₂₄)polyglycosides, les dérivés de N-(alkyl en C₆₋₂₄)glucamine, les oxydes d'amines tels que les oxydes d'(alkyl en C₁₀₋₁₄)amines ou les oxydes de N-(acyl en C₁₀₋₁₄)-aminopropylmorpholine.

Parmi les tensioactifs non ioniques, on utilise de préférence les (alkyl en C₆₋₂₄)polyglycosides, et plus particulièrement les (alkyl en C₈₋₁₈)polyglycosides, les esters d'acides gras du sorbitane polyéthoxylés, les alcools gras polyéthoxylés.

Les tensioactifs amphotères utilisables dans la présente invention peuvent être notamment des dérivés d'amines aliphatiques secondaires ou tertiaires, dans lesquels le groupe aliphatique est une chaîne linéaire ou ramifiée comportant de 8 à 22 atomes de carbone et contenant au moins un groupe anionique tel que, par exemple, un groupe carboxylate, sulfonate, sulfate, phosphate ou phosphonate. On peut citer en particulier les alkyl(C₈₋₂₀)bétaïnes, les sulfobétaïnes, les (alkyl en C₈₋₂₀)amido(alkyl en C₂₋₈)bétaïnes ou les (alkyl en C₈₋₂₀)amido(alkyl en C₂₋₃)sulfobétaïnes. Parmi les dérivés d'amines, on peut citer les produits commercialisés sous la dénomination MIRANOL®, tels que décrits dans les brevets US 2 528 378 et US 2 781 354 et classés dans le dictionnaire CTFA, 3ème édition, 1982, sous les dénominations Amphocarboxyglycinate et Amphocarboxypropionate de structures respectives (1) et (2) :

Rₐ-CONHCH₂CH₂-N(R_{b})(R_{c})(CH₂COO⁻) (1)

dans laquelle :
Rₐ représente un groupe alkyle dérivé d'un acide Rₐ-COOH présent dans l'huile de coprah hydrolysée, un groupe heptyle, nonyle ou undécyle,
R_{b} représente un groupe bêta-hydroxyéthyle, et
R_{c} représente un groupe carboxyméthyle ;
et

Rₐ'-CONHCH₂CH₂-N(B)(C) (2)

dans laquelle :
B représente -CH₂CH₂OX',
C représente -(CH₂)_{z}-Y', avec z = 1 ou 2,
X' représente le groupe -CH₂CH₂-COOH ou un atome d'hydrogène,
Y' représente -COOH ou le groupe -CH₂-CHOH-SO₃H,
Rₐ' représente un groupe alkyle d'un acide Rₐ'-COOH présent dans l'huile de coprah ou dans l'huile de lin hydrolysée, un groupe alkyle saturé ou insaturé en C7-C23, notamment en C₁₇ et sa forme iso, un groupe en C₁₇ insaturé.

Ces composés sont classés dans le dictionnaire CTFA, 5ème édition, 1993, sous les dénominations cocoamphodiacétate de disodium, lauroamphodiacétate de disodium, caprylamphodiacétate de disodium, capryloamphodiacétate de disodium, cocoamphodipropionate de disodium, lauroamphodipropionate de disodium, caprylamphodipro-pionate de disodium, capryloamphodipropionate de disodium, acide lauroamphodipropionique, acide cocoamphodipropionique.
A titre d'exemple, on peut citer le cocoamphodiacétate commercialisé par la société RHODIA sous la dénomination commerciale MIRANOL® C2M concentré.
Parmi les tensioactifs amphotères, on utilise de préférence les (alkyl en C₈₋₂₀)-bétaïnes, les (alkyl en C₈₋₂₀)-amido(alkyl en C₂₋₈)bétaïnes, les alkylamphodiacétates et leurs mélanges.

Les tensioactifs non ioniques et/ou amphotères sont de préférence présents dans la composition utilisée dans le procédé selon l'invention en une quantité allant de 0,1 à 10% en poids, mieux encore de 0,5 à 5% en poids par rapport au poids total de la composition. La quantité totale de tensioactifs dans la composition utilisée dans le procédé selon l'invention va de préférence de 1 à 20% en poids, mieux de 1 à 10% en poids, et encore mieux de 1 à 5% en poids, par rapport au poids total de la composition.

La composition utilisée dans le procédé selon l'invention peut comprendre en outre un ou plusieurs polymères cationiques. Les polymères cationiques utilisables conformément à la présente invention peuvent être choisis parmi tous ceux déjà connus en soi comme améliorant les propriétés cosmétiques des cheveux, à savoir notamment ceux décrits dans la demande de brevet EP-A- 0 337 354 et dans les demandes de brevets français FR-A- 2 270 846, 2 383 660, 2 598 611, 2 470 596 et 2 519 863.
De manière encore plus générale, au sens de la présente invention, l'expression "polymère cationique" désigne tout polymère comprenant des groupements cationiques et/ou des groupements ionisables en groupements cationiques.
Les polymères cationiques préférés sont choisis parmi ceux qui contiennent des motifs comportant des groupements amines primaires, secondaires, tertiaires et/ou quaternaires pouvant soit faire partie de la chaîne principale polymère, soit être portés par un substituant latéral directement relié à celle-ci.
Les polymères cationiques utilisés ont généralement une masse molaire moyenne en nombre ou en poids comprise entre 500 et 5.10⁶ environ, et de préférence comprise entre 10³ et 3.10⁶ environ.
Parmi les polymères cationiques, on peut citer plus particulièrement les polymères du type polyamine, polyaminoamide et polyammonium quaternaire. Ce sont des produits connus.
Les polymères du type polyamine, polyamidoamide, polyammonium quaternaire, utilisables conformément à la présente invention, pouvant être notamment mentionnés, sont ceux décrits dans les brevets français n° 2 505 348 ou 2 542 997.

Parmi ces polymères, on peut citer :
- (1) les homopolymères ou copolymères dérivés d'esters ou d'amides acryliques ou méthacryliques et comportant au moins un des motifs de formules suivantes: 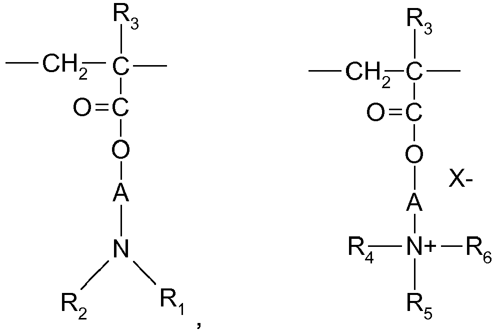 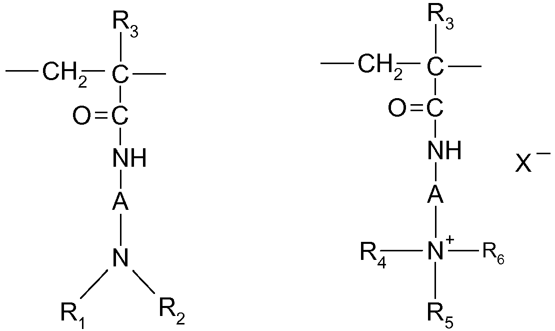 dans lesquelles:
   R3, identiques ou différents, désignent un atome d'hydrogène ou un radical CH3;
   A, identiques ou différents, représentent un groupe alkyle, linéaire ou ramifié, de 1 à 6 atomes de carbone, de préférence 2 ou 3 atomes de carbone ou un groupe hydroxyalkyle de 1 à 4 atomes de carbone ;
   R4, R5, R6, identiques ou différents, représentent un groupe alkyle ayant de 1 à 18 atomes de carbone ou un radical benzyle et de préférence un groupe alkyle ayant de 1 à 6 atomes de carbone;
   R1 et R2, identiques ou différents, représentent hydrogène ou un groupe alkyle ayant de 1 à 6 atomes de carbone et de préférence méthyle ou éthyle;
   X désigne un anion dérivé d'un acide minéral ou organique tel qu'un anion méthosulfate ou un halogénure tel que chlorure ou bromure.
   Les copolymères de la famille (1) peuvent contenir en outre un ou plusieurs motifs dérivant de comonomères pouvant être choisis dans la famille des acrylamides, méthacrylamides, diacétones acrylamides, acrylamides et méthacrylamides substitués sur l'azote par des alkyles inférieurs (C1-C4), des acides acryliques ou méthacryliques ou leurs esters, des vinyllactames tels que la vinylpyrrolidone ou le vinylcaprolactame, des esters vinyliques.
   Ainsi, parmi ces copolymères de la famille (1), on peut citer :
   - les copolymères d'acrylamide et de diméthylaminoéthyl méthacrylate quaternisé au sulfate de diméthyle ou avec un halogénure de diméthyle tels que celui vendu sous la dénomination HERCOFLOC par la société HERCULES,
   - les copolymères d'acrylamide et de chlorure de méthacryloyloxyéthyltriméthylammonium décrit par exemple dans la demande de brevet EP-A-080976 et vendus sous la dénomination BINA QUAT P 100 par la société CIBA GEIGY,
   - le copolymère d'acrylamide et de méthosulfate de méthacryloyloxyéthyltriméthylammonium vendu sous la dénomination RETEN par la société HERCULES,
   - les copolymères vinylpyrrolidone/acrylate ou méthacrylate de dialkylaminoalkyle quaternisés ou non, tels que les produits vendus sous la dénomination "GAFQUAT" par la société ISP comme par exemple "GAFQUAT 734" ou "GAFQUAT 755" ou bien les produits dénommés "COPOLYMER 845, 958 et 937". Ces polymères sont décrits en détail dans les brevets français 2.077.143 et 2.393.573,
   - les terpolymères méthacrylate de diméthylaminoéthyle/vinylcaprolactame/ vinylpyrrolidone tel que le produit vendu sous la dénomination GAFFIX VC 713 par la société ISP,
   - les copolymères vinylpyrrolidone/méthacrylamidopropyldimethylamine commercialisés notamment sous la dénomination STYLEZE CC 10 par ISP.
   - les copolymères vinylpyrrolidone/méthacrylamide de diméthylaminopropyle quaternisé tel que le produit vendu sous la dénomination "GAFQUAT HS 100" par la société ISP,
   - les polymères de préférence réticulés de sels de méthacryloyloxyalkyl(C1-C4) trialkyl(C1-C4)ammonium tels que les polymères obtenus par homopolymérisation du diméthylaminoéthylméthacrylate quaternisé par le chlorure de méthyle, ou par copolymérisation de l'acrylamide avec le diméthylaminoéthylméthacrylate quaternisé par le chlorure de méthyle, l'homo ou la copolymérisation étant suivie d'une réticulation par un composé à insaturation oléfinique, en particulier le méthylène bis acrylamide. On peut plus particulièrement utiliser un copolymère réticulé acrylamide/chlorure de méthacryloyloxyéthyl triméthylammonium (20/80 en poids) sous forme de dispersion comprenant 50% en poids dudit copolymère dans de l'huile minérale. Cette dispersion est commercialisée sous le nom de « SALCARE® SC 92 » par la Société CIBA. On peut également utiliser un homopolymère réticulé du chlorure de méthacryloyloxyéthyl triméthylammonium comprenant environ 50 % en poids de l'homopolymère dans de l'huile minérale ou dans un ester liquide. Ces dispersions sont commercialisées sous les noms de « SALCARE® SC 95 » et « SALCARE® SC 96 » par la Société CIBA.
- (2) les polysaccharides cationiques notamment les celluloses et les gommes de galactomannanes cationiques. Parmi les polysaccharides cationiques, on peut citer plus particulièrement les dérivés d'éthers de cellulose comportant des groupements ammonium quaternaires, les copolymères de cellulose cationiques ou les dérivés de cellulose greffés avec un monomère hydrosoluble d'ammonium quaternaire et les gommes de galactomannanes cationiques.
- (3) les dérivés d'éthers de cellulose comportant des groupements ammonium quaternaires décrits dans le brevet français 1 492 597, et en particulier les polymères commercialisés sous les dénominations "UCARE POLYMER JR" (JR 400 LT, JR 125, JR 30M) ou "LR" (LR 400, LR 30M) par la Société AMERCHOL. Ces polymères sont également définis dans le dictionnaire CTFA comme des ammonium quaternaires d'hydroxyéthylcellulose ayant réagi avec un époxyde substitué par un groupement triméthylammonium.
- (4) les copolymères de cellulose cationiques ou les dérivés de cellulose greffés avec un monomère hydrosoluble d'ammonium quaternaire, sont décrits notamment dans le brevet US4131576, tels que les hydroxyalkyl celluloses, comme les hydroxyméthyl-, hydroxyéthyl- ou hydroxypropyl celluloses greffées notamment avec un sel de méthacryloyléthyl triméthylammonium, de méthacrylmidopropyl triméthylammonium, de diméthyl-diallylammonium.
   Les produits commercialisés répondant à cette définition sont plus particulièrement les produits vendus sous la dénomination "Celquat L 200" et "Celquat H 100" par la Société National Starch.
- (5) les gommes de galactomannane cationiques sont décrites plus particulièrement dans les brevets US 3 589 578 et 4 031 307 en particulier les gommes de guar comprenant des groupements cationiques trialkylammonium. On utilise par exemple des gommes de guar modifiées par un sel (par ex. chlorure) de 2,3-époxypropyl triméthylammonium.
   De tels produits sont commercialisés notamment sous les dénominations commerciales de JAGUAR C13 S, JAGUAR C 15, JAGUAR C 17 ou JAGUAR C162 par la société RHODIA.
- (6) les polymères constitués de motifs pipérazinyle et de radicaux divalents alkylène ou hydroxyalkylène à chaînes droites ou ramifiées, éventuellement interrompues par des atomes d'oxygène, de soufre, d'azote ou par des cycles aromatiques ou hétérocycliques, ainsi que les produits d'oxydation et/ou de quaternisation de ces polymères. De tels polymères sont notamment décrits dans les brevets français 2.162.025 et 2.280.361.
- (7) les polyaminoamides solubles dans l'eau préparés en particulier par polycondensation d'un composé acide avec une polyamine; ces polyaminoamides peuvent être réticulés par une épihalohydrine, un diépoxyde, un dianhydride, un dianhydride non saturé, un dérivé bis-insaturé, une bis-halohydrine, un bis-azétidinium, une bis-haloacyldiamine, un bis-halogénure d'alkyle ou encore par un oligomère résultant de la réaction d'un composé bifonctionnel réactif vis-à-vis d'une bis-halohydrine, d'un bis-azétidinium, d'une bis-haloacyldiamine, d'un bis-halogénure d'alkyle, d'une épilhalohydrine, d'un diépoxyde ou d'un dérivé bis-insaturé ; l'agent réticulant étant utilisé dans des proportions allant de 0,025 à 0,35 mole par groupement amine du polymaoamide ; ces polyaminoamides peuvent être alcoylés ou s'ils comportent une ou plusieurs fonctions amines tertiaires, quaternisées. De tels polymères sont notamment décrits dans les brevets français 2.252.840 et 2.368.508.
- (8) les dérivés de polyaminoamides résultant de la condensation de polyalcoylènes polyamines avec des acides polycarboxyliques suivie d'une alcoylation par des agents bifonctionnels. On peut citer par exemple les polymères acide adipique-diacoylaminohydroxyalcoyldialoylène triamine dans lesquels le radical alcoyle comporte de 1 à 4 atomes de carbone et désigne de préférence méthyle, éthyle, propyle. De tels polymères sont notamment décrits dans le brevet français 1.583.363. Parmi ces dérivés, on peut citer plus particulièrement les polymères acide adipique/diméthylaminohydroxypropyl/diéthylène triamine vendus sous la dénomination "Cartaretine F, F4 ou F8" par la société Sandoz.
- (9) les polymères obtenus par réaction d'une polyalkylène polyamine comportant deux groupements amine primaire et au moins un groupement amine secondaire avec un acide dicarboxylique choisi parmi l'acide diglycolique et les acides dicarboxyliques aliphatiques saturés ayant de 3 à 8 atomes de carbone. Le rapport molaire entre le polyalkylène polylamine et l'acide dicarboxylique étant compris entre 0,8 : 1 et 1,4 : 1; le polyaminoamide en résultant étant amené à réagir avec l'épichlorhydrine dans un rapport molaire d'épichlorhydrine par rapport au groupement amine secondaire du polyaminoamide compris entre 0,5 : 1 et 1,8 : 1. De tels polymères sont notamment décrits dans les brevets américains 3.227.615 et 2.961.347.
   Des polymères de ce type sont en particulier commercialisés sous la dénomination "Hercosett 57" par la société Hercules Inc. ou bien sous la dénomination de "PD 170" ou "Delsette 101" par la société Hercules dans le cas du copolymère d'acide adipique/époxypropyl/diéthylène-triamine.
- (10) les cyclopolymères d'alkyldiallylamine ou de dialkyldiallylammonium tels que les homopolymères ou copolymères comportant comme constituant principal de la chaîne des motifs répondant aux formules (I) ou (II) : 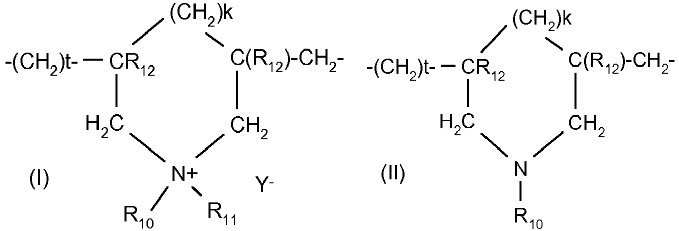 formules dans lesquelles k et t sont égaux à 0 ou 1, la somme k + t étant égale à 1; R12 désigne un atome d'hydrogène ou un radical méthyle ; R10 et R11, indépendamment l'un de l'autre, désignent un groupement alkyle ayant de 1 à 6 atomes de carbone, un groupement hydroxyalkyle dans lequel le groupement alkyle a de préférence 1 à 5 atomes de carbone, un groupement amidoalkyle inférieur (C1-C4) ou R10 et R11 peuvent désigner conjointement avec l'atome d'azote auquel ils sont rattachés, des groupement hétérocycliques, tels que pipéridinyle ou morpholinyle; Y⁻ est un anion tel que bromure, chlorure, acétate, borate, citrate, tartrate, bisulfate, bisulfite, sulfate, phosphate. Ces polymères sont notamment décrits dans le brevet français 2.080.759 et dans son certificat d'addition 2.190.406; R10 et R11, indépendamment l'un de l'autre, désignent de préférence un groupement alkyle ayant de 1 à 4 atomes de carbone.
   Parmi les polymères définis ci-dessus, on peut citer plus particulièrement l'homopolymère de sels (par exemple chlorure) de diméthyldiallylammonium par exemple vendu sous la dénomination "MERQUAT 100" par la société NALCO (et leurs homologues de faibles masses molaires moyenne en poids) et les copolymères de sels (par exemple chlorure) de diallyldiméthylammonium et d'acrylamide commercialisés notamment sous la dénomination "MERQUAT 550" ou "MERQUAT 7SPR".
- (11) les polymères de diammonium quaternaire comprenant des motifs récurrents répondant à la formule : 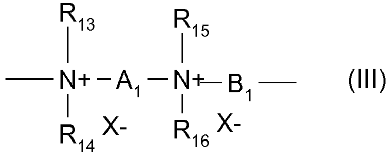 formule (III) dans laquelle :
   R13, R14, R15 et R16, identiques ou différents, représentent des radicaux aliphatiques, alicycliques, ou arylaliphatiques comprenant de 1 à 20 atomes de carbone ou des radicaux hydroxyalkylaliphatiques inférieurs, ou bien R13, R14, R15 et
   R16, ensemble ou séparément, constituent avec les atomes d'azote auxquels ils sont rattachés des hétérocycles comprenant éventuellement un second hétéroatome autre que l'azote ou bien R13, R14, R15 et R16 représentent un radical alkyle en C1-C6 linéaire ou ramifié substitué par un groupement nitrile, ester, acyle, amide ou -CO-O-R17-D ou -CO-NH-R17-D où R17 est un alkylène et D un groupement ammonium quaternaire;
   A1 et B1 représentent des groupements polyméthyléniques comprenant de 2 à 20 atomes de carbone pouvant être linéaires ou ramifiés, saturés ou insaturés, et pouvant contenir, liés à ou intercalés dans la chaîne principale, un ou plusieurs cycles aromatiques, ou un ou plusieurs atomes d'oxygène, de soufre ou des groupements sulfoxyde, sulfone, disulfure, amino, alkylamino, hydroxyle, ammonium quaternaire, uréido, amide ou ester, et
   X⁻ désigne un anion dérivé d'un acide minéral ou organique;
   A1, R13 et R15 peuvent former avec les deux atomes d'azote auxquels ils sont rattachés un cycle pipérazinique; en outre si A1 désigne un radical alkylène ou hydroxyalkylène linéaire ou ramifié, saturé ou insaturé, B1 peut également désigner un groupement (CH2)n-CO-D-OC-(CH2)n-
   dans lequel D désigne :
   un reste de glycol de formule -O-Z-O-, où Z désigne un radical hydrocarboné linéaire ou ramifié ou un groupement répondant à l'une des formules suivantes :
   -(CH2-CH2-O)x-CH2-CH2- ou bien -[CH2-CH(CH3)-O]y-CH2-CH(CH3)-où x et y désignent un nombre entier de 1 à 4, représentant un degré de polymérisation défini et unique ou un nombre quelconque de 1 à 4 représentant un degré de polymérisation moyen ;
   un reste de diamine bis-secondaire tel qu'un dérivé de pipérazine ;
   un reste de diamine bis-primaire de formule : -NH-Y-NH-, où Y désigne un radical hydrocarboné linéaire ou ramifié, ou bien le radical bivalent -CH2-CH2-S-S-CH2-CH2- ;
   un groupement uréylène de formule : -NH-CO-NH- ;
   De préférence, X⁻ est un anion tel que le chlorure ou le bromure.
   Ces polymères ont une masse molaire moyenne en nombre généralement comprise entre 1000 et 100000.
   Des polymères de ce type sont notamment décrits dans les brevets français 2.320.330, 2.270.846, 2.316.271, 2.336.434 et 2.413.907 et les brevets US 2.273.780, 2.375.853, 2.388.614, 2.454.547, 3.206.462, 2.261.002, 2.271.378, 3.874.870, 4.001.432, 3.929.990, 3.966.904, 4.005.193, 4.025.617, 4.025.627, 4.025.653, 4.026.945 et 4.027.020. On peut utiliser plus particulièrement les polymères qui sont constitués de motifs récurrents répondant à la formule : 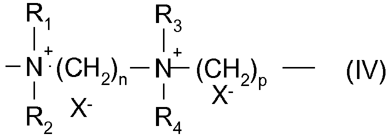 dans laquelle R1, R2, R3 et R4, identiques ou différents, désignent un radical alkyle ou hydroxyalkyle ayant de 1 à 4 atomes de carbone environ, n et p sont des nombres entiers variant de 2 à 20 environ et X⁻ est un anion dérivé d'un acide minéral ou organique.
   Un composé de formule (IV) particulièrement préféré est celui pour lequel R1, R2, R3 et R4 représentent un radical méthyle, n=3, p=6 et X=Cl, dénommé Hexadimethrine chloride selon la nomenclature INCI (CTFA).
- (12) les polymères de polyammonium quaternaires comprenant des motifs de formule (V): 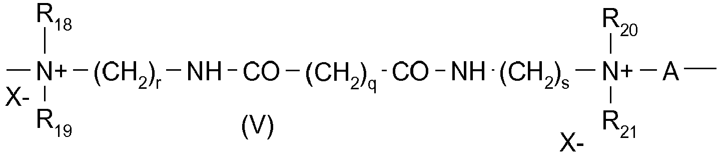 dans laquelle :
   R18, R19, R20 et R21, identiques ou différents, représentent un atome d'hydrogène ou un radical méthyle, éthyle, propyle, β-hydroxyéthyle, β-hydroxypropyle ou -CH2CH2(OCH2CH2)pOH,
   où p est égal à 0 ou à un nombre entier compris entre 1 et 6, sous réserve que R18, R19, R20 et R21 ne représentent pas simultanément un atome d'hydrogène, r et s, identiques ou différents, sont des nombres entiers compris entre 1 et 6,
   q est égal à 0 ou à un nombre entier compris entre 1 et 34,
   X⁻ désigne un anion tel qu'un halogènure,
   A désigne un radical d'un dihalogénure ou de préférence -CH2CH2-O-CH2CH2-. De tels composés sont notamment décrits dans la demande de brevet EP-A-122 324.
   On peut par exemple citer parmi ceux-ci, les produits "Mirapol® A 15", "Mirapol® AD1", "Mirapol® AZ1" et "Mirapol® 175" vendus par la société Miranol.
- (13) les polymères quaternaires de vinylpyrrolidone et de vinylimidazole tels que par exemple les produits commercialisés sous les dénominations Luviquat® FC 905, FC 550 et FC 370 par la société B.A.S.F.
- (14) les polyamines comme le Polyquart® H vendu par COGNIS, référencé sous le nom de "POLYETHYLENEGLYCOL (15) TALLOW POLYAMINE" dans le CTFA.

D'autres polymères cationiques utilisables dans le cadre de l'invention sont des protéines cationiques ou des hydrolysats de protéines cationiques, des polyalkylèneimines, en particulier des polyéthylèneimines, des polymères comprenant des motifs vinylpyridine ou vinylpyridinium, des condensats de polyamines et d'épichlorhydrine, des polyuréylènes quaternaires et les dérivés de la chitine.

Parmi les polymères cationiques mentionnés ci-dessus, on peut utiliser de préférence les dérivés d'éther de cellulose quaternaires tels que les produits vendus sous la dénomination «JR 400» par la Société AMERCHOL, les cyclopolymères cationiques, en particulier les homopolymères ou copolymères de sels (par exemple chlorure) de diméthyldiallylammonium, vendus sous les dénominations « MERQUAT 100 », « MERQUAT 550» et «MERQUAT S» par la société NALCO et leurs homologues de faibles poids moléculaires en poids, les polymères quaternaires de vinylpyrrolidone et de vinylimidazole, les homopolymères ou copolymères éventuellement réticulés de sels de méthacryloy-loxyalkyl(C1-C4) trialkyl(C1-C4)ammonium et leurs mélanges.

S'ils sont présents, les polymères cationiques peuvent être présents à des concentrations allant de 0,01 à 20%, de préférence de 0,05 à 10% et plus particulièrement de 0,1 à 5% en poids, par rapport au poids total de la composition.

La composition utilisée dans le procédé selon l'invention est de préférence aqueuse. Elle peut comprendre de l'eau et éventuellement au moins un solvant organique, notamment choisi parmi les alcools en C₁-C₄ tels que l'éthanol, l'isopropanol, le tertio-butanol ou le n-butanol; les polyols tels que le glycérol, le propylèneglycol et les polyéthylèneglycols.
De préférence, la composition utilisée dans le procédé de l'invention comprend de 70 à 95% en poids d'eau par rapport au poids total de la composition.

Le pH de la composition utilisée dans le procédé selon l'invention est généralement compris entre 2 et 11, de préférence entre 3 et 10 et, mieux encore entre 4 et 8.
La composition utilisée dans le procédé selon l'invention peut comprendre en outre des additifs choisis parmi les polymères anioniques, les polymères non ioniques, les polymères amphotères, les épaississants polymères associatifs ou non associatifs, les épaississants non polymères, les agents nacrants, les opacifiants, les colorants, les pigments, les parfums, les vitamines, les filtres UV, les agents anti-radicalaires, les antipelliculaires, les conservateurs, les agents de stabilisation de pH, les tensioactifs cationiques et leurs mélanges. L'homme du métier veillera à choisir les éventuels additifs et leur quantité de manière à ce qu'ils ne nuisent pas aux propriétés des compositions de la présente invention.
Ces additifs peuvent être présents dans la composition selon l'invention en une quantité allant de 0,01 à 20% en poids par rapport au poids total de la composition.

Les compositions utilisées dans le procédé selon l'invention peuvent être conditionnées sous diverses formes notamment dans des flacons, des flacons-pompe ou dans des récipients aérosols afin d'assurer une application de la composition sous forme vaporisée ou sous forme de mousse. Les compositions utilisées dans le procédé de l'invention peuvent également imprégner des applicateurs, notamment des gants ou des lingettes.

La composition utilisée dans le procédé de l'invention selon l'invention peut être appliquée après une coloration, en particulier une coloration d'oxydation, une permanente, un défrisage, ou tout autre traitement capillaire, en particulier les traitements mettant en oeuvre un ou plusieurs polymères cationiques.
Dans une variante préférée de l'invention, la composition utilisée dans le procédé de l'invention selon l'invention peut être appliquée après une coloration, en particulier une coloration d'oxydation.

La présente invention concerne un procédé de traitement cosmétique, notamment de conditionnement, des fibres kératiniques humaines, et tout spécialement des cheveux, voire des cheveux sensibilisés, dans lequel on applique une composition telle que définie ci-dessus, et on effectue optionnellement un rinçage après un éventuel temps de pose.

Il s'agit tout particulièrement d'un procédé dans lequel l'application de la composition s'effectue après une coloration, en particulier une coloration d'oxydation, une permanente, un défrisage, ou tout autre traitement capillaire, et de préférence après une coloration, en particulier une coloration d'oxydation.

Les exemples suivants sont donnés à titre illustratif de la présente invention. Toutes les quantités indiquées sont exprimées en % en poids de matière commerciale, sauf indication contraire.

### Exemple 1

On a préparé la composition d'après-shampooing suivante :

| COMPOSITION 1 | | |
|---|---|---|
| | Alcool Cétylstéarylique (C16/C18 50/50 en poids) (LANETTE O OR FLAKES de COGNIS) | 7% |
| | Mélange myristate/palmitate/stéarate de myristyle/cétyle/ stéaryle (CRODAMOL MS-V de CRODA) | 1.5% |
| | Cetostearyl sulfate de sodium (C16/C18 50/50) (LANETTE E GRANULES de COGNIS) | 3%MA |
| | Silicone aminée à 20% en poids dans du polydiméthylsiloxane 20 cst (KF 8020 de SHIN ETSU) | 0.5% |
| | Acide Citrique q.s. | pH 5 |
| | Conservateurs | 0.33% |
| | Eau | qsp 100% |
| MA : matière active | | |

### Exemple 2

On a préparé la composition d'après-shampooing suivante :

| COMPOSITION 2 | | |
|---|---|---|
| | Alcool Cétylstéarylique (C16/C18 50/50) (LANETTE O OR FLAKES de COGNIS) | 7% |
| | Mélange Myristate/Palmitate/Stéarate de myristyle/cétyle/ stéaryle (CRODAMOL MS-V de CRODA) | 1,5% |
| | Cetostearyl sulfate de sodium (C16/C18 50/50) (LANETTE E GRANULES de COGNIS) | 3%MA |
| | Silicone aminée à 20% en poids dans du polydimethylsiloxane 20 cst (KF 8020 de SHIN ETSU) | 3,5% |
| | Acide Citrique q.s. | pH 5 |
| | Conservateur | 0,33% |
| | Eau | qsp 100% |

### Exemple 3

On a préparé la composition d'après-shampooing suivante :

| COMPOSITION 3 | | |
|---|---|---|
| | Alcool cétylstéarylique (C16/C18 50/50) (LANETTE O OR FLAKES de COGNIS) | 10% |
| | Mélange Myristate/Palmitate/Stéarate de myristyle/cétyle/stéaryle (CRODAMOL MS-V de CRODA) | 1% |
| | Cétostéaryl sulfate de sodium (C16/C18 50/50) (LANETTE E GRANULES de COGNIS) | 1%MA |
| | Silicone aminée à 20% en poids dans du polydimethylsiloxane 20 cst (KF 8020 de SHIN ETSU) | 3,5% |
| | Acide Citrique q.s. | pH 5 |
| | Conservateur | 0.33% |
| | Eau | qsp 100% |

### Exemple 4

On a préparé la composition d'après-shampooing suivante :

| COMPOSITION 4 | | |
|---|---|---|
| | Alcool cétylstéarylique (C16/C18 50/50) (LANETTE O OR FLAKES de COGNIS) | 10% |
| | Mélange Myristate/Palmitate/Stéarate de myristyle/cétyle/stéaryle (CRODAMOL MS-V de CRODA) | 2% |
| | Cétostéaryl sulfate de sodium (C16/C18 50/50) (LANETTE E GRANULES de COGNIS) | 5%MA |
| | Silicone aminée à 20% en poids dans du polydimethylsiloxane 20 cst (KF 8020 de SHIN ETSU) | 3,5% |
| | Acide Citrique q.s. | pH 5 |
| | Conservateur | 0,33% |
| | Eau | qsp 100% |

### Exemple 5

On a préparé la composition d'après-shampooing suivante :

| COMPOSITION 5 | | |
|---|---|---|
| | Alcool cétylstéarylique (C16/C18 50/50) (LANETTE O OR FLAKES de COGNIS) | 5% |
| | Mélange Myristate/Palmitate/Stéarate de myristyle/cétyle/stéaryle (CRODAMOL MS-V de CRODA) | 1,5% |
| | Cétostéaryl sulfate de sodium (C16/C18 50/50) (LANETTE E GRANULES de COGNIS) | 1%MA |
| | Silicone aminée à 20% en poids dans du polydiméthylsiloxane 20 cst (KF 8020 de SHIN ETSU) | 0,5% |
| | Acide Citrique q.s. | pH 5 |
| | Conservateur | 0,33% |
| | Eau | qsp 100% |

### Exemple 6

On a préparé la composition d'après-shampooing suivante :

| COMPOSITION 6 | | |
|---|---|---|
| | Alcool cétylstéarylique (C16/C18 50/50) (LANETTE O OR FLAKES de COGNIS) | 10% |
| | Mélange Myristate/Palmitate/Stéarate de myristyle/cétyle/stéaryle (CRODAMOL MS-V de CRODA) | 1% |
| | Cétostéaryl sulfate de sodium (C16/C18 50/50) (LANETTE E GRANULES de COGNIS) | 1%MA |
| | Diméthicone (DOW CORNING 200 FLUID 500000 cst de DOW CORNING) | 0,65% |
| | Diméthicone (MIRASIL DM 50 de BLUESTAR) | 1,95% |
| | Acide citrique q.s. | pH 5 |
| | Conservateur | 0,33% |
| | Eau | qsp 100% |

### Exemple 7

On a préparé la composition d'après-shampooing suivante :

| COMPOSITION 7 | | |
|---|---|---|
| | Alcool cétylstéarylique (C16/C18 50/50) (LANETTE O OR FLAKES de COGNIS) | 10% |
| | Mélange Myristate/Palmitate/Stéarate de myristyle/cétyle/stéaryle (CRODAMOL MS-V de CRODA) | 2% |
| | Cétostéaryl sulfate de sodium (C16/C18 50/50) (LANETTE E GRANULES de COGNIS) | 5%MA |
| | Diméthicone (DOW CORNING 200 FLUID 500000 cst de DOW CORNING) | 0,65% |
| | Diméthicone (MIRASIL DM 50 de BLUESTAR) | 1,95% |
| | Acide citrique q.s. | pH 5 |
| | Conservateur | 0,33% |
| | Eau | qsp 100% |

### Exemple 8

On a préparé la composition d'après-shampooing suivante :

| COMPOSITION 8 | | |
|---|---|---|
| | Alcool cétylstéarylique (C16/C18 50/50) (LANETTE O OR FLAKES de COGNIS) | 5% |
| | Mélange Myristate/Palmitate/Stéarate de myristyle/cétyle/stéaryle (CRODAMOL MS-V de CRODA) | 1,5% |
| | Cétostéaryl sulfate de sodium (C16/C18 50/50) (LANETTE E GRANULES de COGNIS) | 1%MA |
| | Diméthicone (DOW CORNING 200 FLUID 500000CS de DOW CORNING) | 0,65% |
| | Diméthicone (MIRASIL DM 50 de BLUESTAR) | 1,95% |
| | Acide citrique q.s. | pH 5 |
| | Conservateur | 0.33% |
| | Eau | qsp 100% |

### Exemple 9

On a préparé la composition d'après-shampooing suivante :

| COMPOSITION 9 | | |
|---|---|---|
| | Alcool cétylstéarylique (C16/C18 50/50) (LANETTE O OR FLAKES de COGNIS) | 5% |
| | Mélange Myristate/Palmitate/Stéarate de myristyle/cétyle/stéaryle (CRODAMOL MS-V de CRODA) | 1,5% |
| | Cétostéaryl sulfate de sodium (C16/C18 50/50) (LANETTE E GRANULES de COGNIS) | 1%MA |
| | Octyldodécanol | 2% |
| | Acide citrique q.s. | pH 5 |
| | Conservateur | 0,33% |
| | Eau | qsp 100% |

Les compositions des exemples 1 à 9 sont stables. Si on remplace le cétostéaryl-sulfate de sodium par du laurylsulfate de sodium dans la composition 1, la composition n'est pas stable.
Les cheveux humides et séchés, traités avec les compositions des exemples 1 à 9 présentent de très bonnes propriétés cosmétiques, notamment en terme de lissage.

## Revendications

1. Procédé de traitement cosmétique, notamment de conditionnement, des fibres kératiniques humaines, et tout spécialement des cheveux, voire des cheveux sensibilisés, dans lequel on applique sur lesdites fibres kératiniques, une composition cosmétique non colorante et non oxydante, comprenant :
- un ou plusieurs tensioactifs anioniques sulfates, sulfonates ou carboxyliques, tels qu'au moins 50% en poids d'entre eux comportent des chaînes grasses ayant un nombre d'atomes de carbone supérieur ou égal à 14 ; présents dans la composition en une quantité totale allant de 0,5 à 10% en poids, par rapport au poids total de la composition ;
les tensioactifs anioniques sulfates, sulfonates ou carboxyliques étant choisis parmi les sels des types suivants : les alkylsulfates, les alkyléthersulfates, les alkylamidoéthersulfates, les alkylarylpolyéthersulfates, les monoglycéride-sulfates; les alkylsulfonates, les alkylamidesulfonates, les alkylarylsulfonates, les alpha-oléfine-sulfonates, les paraffine-sulfonates, les alkylsulfoacétates, les groupes alkyle et acyle de tous ces composés comportant de préférence de 14 à 30 atomes de carbone, mieux de 16 à 22 atomes de carbone, et le groupe aryle désignant de préférence un groupe phényle ou benzyle;
- un ou plusieurs alcools gras solides, présents en une quantité allant de 4 à 10% en poids, par rapport au poids total de la composition, et
- un ou plusieurs esters gras solides de monoalcools,
et on effectue optionnellement un rinçage après un éventuel temps de pose.

2. Procédé selon la revendication 1, dans lequel les tensioactifs anioniques sont choisis parmi :
- les alkylsulfates en C16-C18 et les alkyléthersulfates en C16-18; en particulier sous forme de sels de métaux alcalins ou alcalino-terreux, d'ammonium, d'amine ou d'aminoalcool.

3. Procédé selon l'une des revendications précédentes, dans lequel les tensioactifs anioniques sont présents dans la composition en une quantité totale allant de 1 à 5% en poids par rapport au poids total de la composition.

4. Procédé selon l'une des revendications précédentes, dans lequel les alcools gras solides sont de structure R-OH, avec R désignant un groupe alkyle linéaire, éventuellement substitué par un ou plusieurs groupes hydroxy, comprenant de 12 à 40, mieux de 12 à 34, voire de 12 à 30, et tout préférentiellement de 12 à 24, atomes de carbone.

5. Procédé selon l'une des revendications précédentes, dans lequel les alcools gras solides sont choisis parmi l'alcool cétylique, l'alcool stéarylique, l'alcool béhénylique et leurs mélanges, tels que l'alcool cétylstéarylique.

6. Procédé selon l'une des revendications précédentes, dans lequel les esters gras solides de monoalcools sont des esters gras solides de monoalcools comportant au moins 10 atomes de carbone, encore mieux de monoalcools saturés comportant au moins 10 atomes de carbone.

7. Procédé selon l'une des revendications précédentes, dans lequel les esters gras solides sont choisis parmi les myristates de myristyle, de cétyle ou de stéaryle, les palmitates de myristyle, de cétyle ou de stéaryle, les stéarates de myristyle, de cétyle ou de stéaryle, et leurs mélanges.

8. Procédé selon l'une des revendications précédentes, dans lequel les esters gras solides sont présents en une quantité comprise entre 0,1 et 10% en poids, de préférence entre 0,5 et 5% en poids, par rapport au poids total de la composition.

9. Procédé selon l'une des revendications précédentes, dans lequel la composition comprend en outre au moins un corps gras liquide choisi parmi les alcools gras liquides, les esters gras liquides, les huiles de silicone, les hydrocarbures en C6-C16, les hydrocarbures à plus de 16 atomes de carbone, les huiles non siliconées d'origine animale, les huiles végétales de type triglycérides, les triglycérides synthétiques, les huiles fluorées, et leurs mélanges.

10. Procédé selon la revendication 9, dans lequel les corps gras liquides sont choisis parmi les alcools gras liquides et les huiles de silicone, de préférence les huiles de silicone.

11. Procédé selon l'une des revendications 9 à 10, dans lequel les corps gras liquides sont présents en une quantité allant de 0,01 à 20%, de préférence de 0,1 à 10%, et mieux de 0,5 à 5% en poids, par rapport au poids total de la composition.

12. Procédé selon l'une des revendications précédentes, dans lequel la composition comprend en outre au moins un tensioactif non ionique et/ou au moins un tensioactif amphotère.

13. Procédé selon l'une des revendications précédentes, dans lequel la composition comprend en outre au moins un polymère cationique.

14. Procédé selon l'une des revendications précédentes, dans lequel la composition comprend de l'eau et éventuellement au moins un solvant organique.

15. Procédé selon l'une des revendications précédentes, dans lequel l'application de la composition s'effectue après une coloration, en particulier une coloration d'oxydation, une permanente, un défrisage, ou tout autre traitement capillaire, et de préférence après une coloration, en particulier une coloration d'oxydation.

## Patentansprüche

1. Verfahren zur kosmetischen Behandlung, insbesondere Konditionierung, menschlicher Keratinfasern und speziell Haare, sogar empfindlicher Haare, bei dem man auf diese Keratinfasern eine nicht färbende und nicht oxidierende kosmetische Zusammensetzung aufbringt, umfassend:
- ein oder mehrere anionische Sulfat-, Sulfonat- oder Carbonsäuretenside derart, dass mindestens 50 Gew.-% unter ihnen Fettketten mit einer Anzahl an Kohlenstoffatomen von mehr als oder gleich 14 umfassen; die in der Zusammensetzung in einer Gesamtmenge von 0,5 bis 10 Gew.-%, bezogen auf das Gesamtgewicht der Zusammensetzung, vorliegen;
wobei die anionischen Sulfat-, Sulfonat- und Carbonsäuretenside aus Salzen der folgenden Typen ausgewählt sind: Alkylsulfaten, Alkylethersulfaten, Alkylamidoethersulfaten, Alkylarylpolyethersulfaten, Monoglyceridsulfaten, Alkylsulfonaten, Alkylamidsulfonaten, Alkylarylsulfonaten, alpha-Olefinsulfonaten, Paraffinsulfonaten, Alkylsulfoacetaten, wobei die Alkyl- und Acylgruppen dieser sämtlichen Verbindungen vorzugsweise 14 bis 30 Kohlenstoffatome, besser 16 bis 22 Kohlenstoffatome, enthalten und die Arylgruppe vorzugsweise für eine Phenyl- oder Benzylgruppe steht;
- einen oder mehrere feste Fettalkohole, der bzw. die in einer Menge von 4 bis 10 Gew.-%, bezogen auf das Gesamtgewicht der Zusammensetzung, vorliegt bzw. vorliegen, und
- einen oder mehrere feste Monoalkoholfettester,
und man gegebenenfalls eine Spülung nach einer eventuellen Einwirkzeit durchführt.

2. Verfahren nach Anspruch 1, wobei die anionischen Tenside aus den Folgenden ausgewählt sind:
- C16-C18-Alkylsulfaten und C16-C18-Alkylethersulfaten, insbesondere in Form von Alkali- oder Erdalkalimetall-, Ammonium-, Amin- oder Aminoalkoholsalzen.

3. Verfahren nach einem der vorhergehenden Ansprüche, wobei die anionischen Tenside in der Zusammensetzung in einer Gesamtmenge von 1 bis 5 Gew.-%, bezogen auf das Gesamtgewicht der Zusammensetzung, vorliegen.

4. Verfahren nach einem der vorhergehenden Ansprüche, wobei die festen Fettalkohole die Struktur R-OH haben, wobei R eine gerade Alkylgruppe darstellt, die gegebenenfalls mit einer oder mehreren, 12 bis 40, besser 12 bis 34, insbesondere 12 bis 30 und besonders bevorzugt 12 bis 24 Kohlenstoffatome umfassenden Hydroxylgruppen substituiert ist.

5. Verfahren nach einem der vorhergehenden Ansprüche, wobei die festen Fettalkohole aus Cetylalkohol, Stearylalkohol, Behenylalkohol und deren Gemischen, wie Cetylstearylalkohol, ausgewählt sind.

6. Verfahren nach einem der vorhergehenden Ansprüche, wobei es sich bei den festen Monoalkoholfettestern um feste Fettester von Monoalkoholen mit mindestens 10 Kohlenstoffatomen, noch besser gesättigten Monoalkoholen mit mindestens 10 Kohlenstoffatomen, handelt.

7. Verfahren nach einem der vorhergehenden Ansprüche, wobei die festen Fettester aus Myristyl-, Cetyl- oder Stearylmyristat, Myristyl-, Cetyl- oder Stearylpalmitat, Myristyl-, Cetyl- oder Stearylstearat und deren Gemischen ausgewählt sind.

8. Verfahren nach einem der vorhergehenden Ansprüche, wobei die festen Fettester in einer Menge zwischen 0,1 und 10 Gew.-%, vorzugsweise zwischen 0,5 und 5 Gew.-%, bezogen auf das Gesamtgewicht der Zusammensetzung, vorliegen.

9. Verfahren nach einem der vorhergehenden Ansprüche, wobei die Zusammensetzung außerdem mindestens einen flüssigen Fettkörper umfasst, der ausgewählt ist aus flüssigen Fettalkoholen, flüssigen Fettestern, Silikonölen, C6-C16-Kohlenwasserstoffen, Kohlenwasserstoffen mit mehr als 16 Kohlenstoffatomen, Nicht-Silikon-Ölen tierischen Ursprungs, Pflanzenölen des Triglyceridtyps, synthetischen Triglyceriden, fluorierten Ölen und deren Gemischen.

10. Verfahren nach Anspruch 9, wobei die flüssigen Fettkörper aus flüssigen Fettalkoholen und Silikonölen, vorzugsweise Silikonölen, ausgewählt sind.

11. Verfahren nach einem der Ansprüche 9 bis 10, wobei die flüssigen Fettkörper in einer Menge von 0,01 bis 20 Gew.-%, vorzugsweise von 0,1 bis 10 Gew.-% und besser von 0,5 bis 5 Gew.-%, bezogen auf das Gesamtgewicht der Zusammensetzung, vorliegen.

12. Verfahren nach einem der vorhergehenden Ansprüche, wobei die Zusammensetzung außerdem mindestens ein nichtionisches Tensid und/oder mindestens ein amphoteres Tensid umfasst.

13. Verfahren nach einem der vorhergehenden Ansprüche, wobei die Zusammensetzung außerdem mindestens ein kationisches Polymer umfasst.

14. Verfahren nach einem der vorhergehenden Ansprüche, wobei die Zusammensetzung außerdem Wasser und gegebenenfalls mindestens ein organisches Lösungsmittel umfasst.

15. Verfahren nach einem der vorhergehenden Ansprüche, wobei das Aufbringen der Zusammensetzung nach einer Koloration, insbesondere einer oxidativen Koloration, einer Dauerwelle, einer Glättung oder jeder anderen Haarbehandlung und vorzugsweise nach einer Koloration, insbesondere einer oxidativen Koloration, erfolgt.

## Claims

1. Cosmetic treatment process, notably for conditioning human keratin fibres, and most especially the hair, or even sensitized hair, in which a non-colouring and non-oxidizing cosmetic composition is applied to said keratin fibres, said composition comprising:
- one or more sulfate, sulfonate or carboxylic anionic surfactants, such that at least 50% by weight thereof include fatty chains containing a number of carbon atoms of greater than or equal to 14; present in the composition in a total amount ranging from 0.5% to 10% by weight, relative to the total weight of the composition;
the sulfate, sulfonate or carboxylic anionic surfactants being chosen from salts of the following types: alkyl sulfates, alkyl ether sulfates, alkylamido ether sulfates, alkylaryl polyether sulfates, monoglyceride sulfates, alkylsulfonates, alkylamidesulfonates, alkylarylsulfonates α-olefin sulfonates, paraffin sulfonates, alkylsulfoacetates, the alkyl and acyl groups of all these compounds preferably comprising from 14 to 30 carbon atoms and better still from 16 to 22 carbon atoms, and the aryl group preferably denoting a phenyl or benzyl group;
- one or more solid fatty alcohols, present in an amount ranging from 4% to 10% by weight, relative to the total weight of the composition; and
- one or more solid fatty esters of monoalcohols;
and rinsing is optionally performed after an optional leave-on time.

2. Process according to Claim 1, in which the anionic surfactants are chosen from:
- C16-C18 alkyl sulfates and C16-C18 alkyl ether sulfates, in particular in the form of alkali metal, alkaline-earth metal, ammonium, amine or amino alcohol salts.

3. Process according to one of the preceding claims, in which the anionic surfactants are present in the composition in a total amount ranging from 1% to 5% by weight relative to the total weight of the composition.

4. Process according to one of the preceding claims, in which the solid fatty alcohols are of structure R-OH with R denoting a linear alkyl group, optionally substituted with one or more hydroxyl groups, comprising from 12 to 40, better still from 12 to 34, or even from 12 to 30, and most preferentially from 12 to 24 carbon atoms.

5. Process according to one of the preceding claims, in which the solid fatty alcohols are chosen from cetyl alcohol, stearyl alcohol, behenyl alcohol and mixtures thereof such as cetylstearyl alcohol.

6. Process according to one of the preceding claims, in which the solid fatty esters of monoalcohols are solid fatty esters of monoalcohols comprising at least 10 carbon atoms, and better still of saturated monoalcohols comprising at least 10 carbon atoms.

7. Process according to one of the preceding claims, in which the solid fatty esters are chosen from myristyl myristate, cetyl myristate, stearyl myristate, myristyl palmitate, cetyl palmitate, stearyl palmitate, myristyl stearate, cetyl stearate and stearyl stearate, and mixtures thereof.

8. Process according to one of the preceding claims, in which the solid fatty esters are present in an amount of between 0.1% and 10% by weight and preferably between 0.5% and 5% by weight, relative to the total weight of the composition.

9. Process according to one of the preceding claims, in which the composition also comprises at least one liquid fatty substance chosen from liquid fatty alcohols, liquid fatty esters, silicone oils, C6-C16 hydrocarbons, hydrocarbons containing more than 16 carbon atoms, non-silicone oils of animal origin, plant oils of triglyceride type, synthetic triglycerides and fluoro oils, and mixtures thereof.

10. Process according to Claim 9, in which the liquid fatty substances are chosen from liquid fatty alcohols and silicone oils, preferably silicone oils.

11. Process according to either of Claims 9 and 10, in which the liquid fatty substances are present in an amount ranging from 0.01% to 20% by weight, preferably from 0.1% to 10% by weight and better still from 0.5% to 5% by weight relative to the total weight of the composition.

12. Process according to one of the preceding claims, in which the composition also comprises at least one nonionic surfactant and/or at least one amphoteric surfactant.

13. Process according to one of the preceding claims, in which the composition also comprises at least one cationic polymer.

14. Process according to one of the preceding claims, in which the composition comprises water and optionally at least one organic solvent.

15. Process according to one of the preceding claims, in which the application of the composition is performed after dyeing, in particular oxidation dyeing, permanent-waving or relaxing the hair, or any other haircare treatment, and preferably after dyeing, in particular oxidation dyeing.
